# EUROPÄISCHE PATENTSCHRIFT

(11) **EP 1 017 843 B1**
(45) Veröffentlichungstag und Bekanntmachung des Hinweises auf die Patenterteilung: **01.08.2001**
(21) Anmeldenummer: 98951475.7
(22) Anmeldetag: 26.09.1998
(51) Int. Cl.: C12Q 1/02, C12N 15/62

(54) **VERFAHREN ZUR BESTIMMUNG VON WIRKSUBSTANZEN**
METHOD FOR DETERMINING ACTIVE AGENTS
PROCEDE POUR DETERMINER LA PRESENCE DE PRINCIPES ACTIFS

(30) Priorität: 27.09.1997 EP 97116840; 29.10.1997 EP 97118756
(43) Veröffentlichungstag der Anmeldung: 12.07.2000
(73) Patentinhaber: Evotec BioSystems AG, 22525 Hamburg (DE)
(72) Erfinder: STRAUSS, Andreas, D-22525 Hamburg (DE); THUMM, Günther, D-22525 Hamburg (DE); POHLNER, Johannes, D-22525 Hamburg (DE); GÖTZ, Friedrich, D-22525 Hamburg (DE)
(74) Vertreter: Meyers, Hans-Wilhelm, Dr.Dipl.-Chem.
(86) Internationale Anmeldenummer: EP9806137
(87) Internationale Veröffentlichungsnummer: WO9916894

(56) Entgegenhaltungen:
- WO-A-96/34976
- WO-A-97/08553
- US-A- 5 616 686
- HUNG TON-THAT: "Anchor structure of Staphylococcal surface proteins" THE JOURNAL OF BIOLOGICAL CHEMISTRY, Bd. 272, Nr. 35, August 1997, Seiten 22285-22292, XP002059735
- SCHNEEWIND O ET AL: "SORTING OF PROTEIN A TO THE STAPHYLOCOCCAL CELL WALL" CELL, Bd. 70, 24. Juli 1992, Seiten 267-281, XP002017666 in der Anmeldung erwähnt
- SCHNEEWIND, O. ET AL.: "Structure of the cell wall anchor of surface proteins in Staphylococcus aureus" SCIENCE, Bd. 268, 7. April 1995, Seiten 103-106, XP002059736 in der Anmeldung erwähnt
- SAMUELSON P ET AL: "CELL SURFACE DISPLAY OF RECOMBINANT PROTEINS ON STAPHYLOCOCCUS CARNOSUS" JOURNAL OF BACTERIOLOGY, Bd. 177, Nr. 6, März 1995, Seiten 1470-1476, XP000612670
- MURAKAMI, Y. ET AL.: "Role of the charged tail in localasation of a surface protein antigen in Streptococcus mutans" INFECT. IMMUN., Bd. 65, Nr. 4, April 1997, Seiten 1531-1523, XP002059737

### SEQUENZPROTOKOLL

(1) ALLGEMEINE ANGABEN:
   (i) ANMELDER:
      (A) NAME: Evotec BioSystems GmbH
      (B) STRASSE: Grandweg 64
      (C) ORT: Hamburg
      (E) LAND: Deutschland
      (F) POSTLEITZAHL: D-22529
   (ii) BEZEICHNUNG DER ERFINDUNG: Verfahren zur Bestimmung von Wirksubstanzen
   (iii) ANZAHL DER SEQUENZEN: 15
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 604 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 1:
   ACCCATGTTG GAAAGAATCA TATTCTCCAT GTAAATCACT GACAAAGTGT TCAGTGCCTT     60
   TTGTTAGATT CTAGACTGAT GATTTCTGTA GCGAGTTGTT CTTCGTGATT AAATTTTTCT    120
   GCAAGTAAGT CAAAGTATTT ATGTTTAAGT TCACGATCTG TGGTCTTCAT TTCTTCCTCC    180
   TGTGCTTCTA TATTAATAGC GCTTACATTT GATTATTGTA CTTTTTTATA AAATTTTCAA    240
   TGGTAGCGGT TAAATTGTTG CAAATTAGTT GCATTAAAAG TAAACGATTT CAAATTTAGG    300
   TGAGAATTAG GTAGTATTTA CTATAATCTG AGACAATCTC CTAGAAACAT GTGATATTAT 360
   TATTTTTGAA TACAATATTT TWACATCAGG AGGCATTATG ACATCCCTAT TTTCTGTAGA 420
   GCAATTGAAC AAATCTTTCA AAGACAGCAC TTTTAAAATA AATAATGTGT CATTTGAAGT 480
   GCATGAAGGT GAAATTGTAG CGTTTTTCGG CCAGAACGGC TCTGGCAAAT CTACTTTGAT 540
   TCGTATGATT GTGGGTGATT ATCCCTTGTT CCAGGGAAAT TGTTTTTTTT KGTGNNGCGG 600
   TGGG 604
(2) ANGABEN ZU SEQ ID NO: 2:
   (i) SEQUENZKENNZEICHEN:
      (A) LÄNGE: 295 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 2:
   CTAAAAAGGA GGATGCCAAC TATGAATAAA TTTGACAAAG CATATGAGAT TTGTGCAAAG 60
   AGATTCTTGA AATCGGAAAT GAACGTGACG ATCGTACACA TACAGGTACA ATTTCAAAGT 120
   TCGGCCACCA ATTACGGTTT GATTTATCTG AAGGATTTCC ATTACTTACT ACAAAAAAAG 180
   TTTCGTTTAA ATTATTAGCT ACTGAACTCG TTTGGTTCAT AAAAGGTGAT ACTAAGATCA 240
   AGTACTTACT TCAATATAGT AATAATATCC GGAATGAGTG GGCATTTGAA AAATA 295
(2) ANGABEN ZU SEQ ID NO: 3:
   (i) SEQUENZKENNZEICHEN:
      (A) LÄNGE: 429 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 3:
   CTATTATAAT CAATATTAAC AAGCCAGCAA GCGGAAAGCA AGCGGCTTAT TATAAATTAG 60
   CAAGAGGCCA TCCAAAAATG CTAAAGCAAT ATATACCAAT TAGTTGTCAC TTCTTCGTTA 120
   GGTATTCTGC TACTVTCTCG CATCAAATGG TATCTTTTTA TATTCTATTC CTGACTGGTT 180
   TAGCAGCTGA ATTKCATATT CACGACTTAT GATAATCTTG AGCATAATAT ATTCCTTTAA 240
   TTCCAGCCTG AATTATTGAT TACGTACAAC TTAAACAAGG AAAATGCGCT ACACATATGG 300
   CTGCACCTTC AGTAGAAACA CCCTGCTTTT GTACATCGCA AAAAGGCGCG CACCTCAGCA 360
   CGTACTGCTC AACACAACGA CTATCCTCCA CGAGGCATCC TCTACAATCA CAGTGAACCA 420
   CCTGCTACG 429
(2) ANGABEN ZU SEQ ID NO: 4:
   (i) SEQUENZKENNZEICHEN:
      (A) LÄNGE: 748 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 4:
   KCGGGAGCCT AATACTAAAA ATAATGAATT CAATGTCGAA AATTAAACTG AATGCATTAC 60
   GTGAGCAACG TCATATATTA AATGAATTGG TAGATGAAGG AGAAGTAAGT GAAAAAACGG 120
   CACTGAAAGT AAGAGAAGAG GTTAATTATG ATGAAATGAT TTTAGTAGAG TCGCTTAACT 180
   TAATATATTA AATAAGCTGT AAAACAGATG CTTAGCGACG ATTGTTTTGC AGCTTTTTTA 240
   TGTAAAAMAG GAGCCAGTCT TGGAGGGGAC TGGCTCTTTG AAAAGCGATA TCATCGATTA 300
   CCGCTTTTTC CTTAGGGGTT TTTGTGGTCT CTAGGGAGGA TTACGAGCAC TGCTTATTAC 360
   TTATGTTTAG GATAGATTAT TTATGGGAGA TGACGGTATT TGAATTAGGA GGTGAACCGG 420
   CATCTTAAGG ATAATGTGTT ATTTCATACT GTTGTGGACA GTTTCAATAT TAGATTCCMC 480
   CATTTTGTAM TAGGAATCGC CCTTACTGCC TTTTTTGCCA ATAGAGTCTG TATAAACTTC 540
   TCCMGAAATC GGTACACCTG TTTCTTCACY TAAACYYTGC RGGACTTTTG TGGGGCCCCA 600
   CCTGGGGTYT CGCGCCAAAA AGGAGGGTTT TAAGGGAAGG GATTTTACAA ATTGAANCGC 660
   CTGTTCAACT TGTTCGGTGT CCCCGTTTTT CCGAATTGAG TTCCACAATA TAGCGGGTGT 720
   AATGCCCTAT TTGTTTAGAG AAATATTT 748
(2) ANGABEN ZU SEQ ID NO: 5:
   (i) SEQUENZKENNZEICHEN:
      (A) LÄNGE: 572 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 5:
   GGCCTATAAG TATGGACAGT CACTTGTTTA GAGAAAATTT TATAATTTTG TAAATCAATG 60
   ACTAATTTTG CAACATCTTT AGGTGCCATA TATAAATTTC CAGCTCCATA GTATTGATCT 120
   AGAAACTCAG GCTTTTGGAA AATCTTCATA TTATTATATC CCTGTGCCAT ATAAGGTTGG 180
   AAAGTTGTGT CGTTATAAAA AGCGGTTCTT TCTAAATCAT ATGGCTTCGC AAAGTTATCA 240
   TTAAAATATT TTGCATAAGA TTCTTTTGTA ACAACTTCTA TTACGCGTGC CAAAACAATA 300
   TAATTTCCAT CATTATACAT ATGCTTAGTG ATACGTACCA GGCTGAATAC CTTCATTTTG 360
   CATCCATTTC GATGCGCCAT CTATGTCATG AATTTGACTT GTAGCTTTAT ATTTTTTCAA 420
   CCCTGTTCTA TGAAGCATAA AGTCTTTTAA ATAGAGTGGT TTCGCTCGTC TTAAACCATG 480
   GAAGATATTT TGTGATTGGA TCATTTATAT TCACTTTTGC TTCAGTTTCT AATTTTAATC 540
   AACATCAGGG CCAGTCGTAA ATTTTTTGGG GC 572
(2) ANGABEN ZU SEQ ID NO: 6:
   (i) SEQUENZKENNZEICHEN:
      (A) LÄNGE: 690 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 6:
   CTAAAAAACA ATAAAADGTG CAGTGTAGAA AATTAATTCT AGCGACTGAC TTTTTTATTA 60
   TGATGCTTTA TCATTGAAAA TTGCATTAAG GTTAAAGAAT TTAGTTAATG CATTTCCGCT 120
   TTGTTGATTA TTATTGCCTT GGTATTATTA CCTTGTTGAT TATTATTGCC TTGGAGTTAT 180
   TACCTTGTTG ATTGCTTTGT CCAGAACTAT TATTTGAACC ACTGTTACTA CTGCTGCTGC 240
   CACCATTTAC AGTACCGTTA CTATTAACTT TTCTATTAGT AGTATCGTTG TCTGGGTGTC 300
   CAGCTACAGA TAAATCTTCT TTGCTTGAAC CATCTACAGA AGAAGGTTTT TTGAAGTCTG 360
   CACCGTCACG AGGACTAATG TCTGACATTA CAATCTTCAA GTAAATATTG TGGATAATCT 420
   TGTTCACTAT GACCAAACAA ATGAATTTTC ACCATAATTG TTTTACTTTA TTGAAAGCCA 480
   TCCAAAACTG ACATTGAATA TTTAGGTGTG AAACAATTAA TCCAACATCT TTGGCTGCCG 540
   TCATCGGTAA GTTGTATTGT TGAAAATGTT TCACTACCGT AAGTAACCTG TCCCAGTTTT 600
   TGCTGCTAGA TTAACAACCT GAAACGCCGT GTCCCAAAAA AGCAGAACCC CAAGCTTCAA 660
   AAGTACCTTT TAAAGAAATT CCTGAAAGGC 690
(2) ANGABEN ZU SEQ ID NO: 7:
   (i) SEQUENZKENNZEICHEN:
      (A) LÄNGE: 459 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 7:
   CATAATAAGG TCGATACTCA GGGTTTTGTA TTAACTCTTT TTGTGAATGT CCTCAAATTT 60
   TCCTAGTGAA CGTTCTCTTA ACAATGCTGT AATAGTAACA GGAATAGTGT TATCAAAACC     120
   ATTTTCATAG GTCTGTACAG TCCTCTTTAA ATCTGAAACA TAAATATGGT CAATAGGAAT     180
   ATCTGAAAAG TATTCTTTCA ATTTTTTTGC TGACTTGATA CCTGTTTCAG TCAAAGCAAC     240
   ATTTAGTTGA CCGCAAAAGT AATCTTGCCC GTGTTTATTA TCATAATTTG CTGTCGATTC     300
   TCCGTCTCGA ATTAAATAAA TCTCCAAGTT GTCTCACTCC AATTATTAAC TTACTTTCAG     360
   GCCAGTTACT TTACTGATAT CTTTATWWAG ATAATAAAGT TATAGACACC ACTAAATATT     420
   TTATTCAACA GTATGATAAC ACGGTTTTWA TCAATATAT                            459
(2) ANGABEN ZU SEQ ID NO: 8:
   (i) SEQUENZKENNZEICHEN:
      (A) LÄNGE: 800 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 8:
   CCAGATTAGA AGTATATACC AGAAGAAGCT GAGAAGCACC TGGTACTCTT GTTGGGTATG      60
   CCGATAATTA CATGAAAGTT GAATTTGAAG GTGACGATTC ATTAATAGGA GAGTTGGTTC     120
   GTGTTAAAAT TACACAAGCT GGATATCCAT TGAATCAAGG ACATGTAGTT MKAGTTATTG     180
   ACCATGCATC TAACCGTAGT GAATTTACTG CTATTATTTA AGATTCAAAA AATTAATTAT     240
   TCTAATTGAC CCCAACTATC ATATATATTA TACTTATGAA TGCGTCATAT TTAGTTATGC     300
   TATGTATATG AAAATAAAGT TCCGTTGATA TTTGGAGGGA GGGAAATACA GATGTCTAAA     360
   ACMGTAGTCC GTAAAAACGA ATCACTTGAA GATGCTTTAC GCCGTTTCAA ACGCTCAGTT     420
   TCAAAAAGCG GTACAATTCA AGAAGTACGT AAACGTGAAT TTTACGAAAA ACCAAGTGTT     480
   AAACGTAAAA AGAAATCAGA AGCTGCACGT AAACGTAAAT TTAAATAATT GATATGTCTG     540
   TTGACTCCCT CAACAACAAA TATGAATTAT ATAAATGCCG TTTTTCGAAG GTCATATATA     600
   TTACCAATTC AGGTAGTATT TATGGCCTTT TTTCTTCTCA TTTTAAAATC AAAATATATT 660
   TTCTATAAAT TCACTCTTTT ATAACTATAA TTCCTTTAAG TAGTCMTGTA TAATGAAATG 720
   AGAGCGAGGT GAATTTTTGT CTCATCCCAC CTGGGGATTT TTACCTAATA ATTTCCGTGT 780
   TTCAGACTAG TCNAAGCCCC 800
(2) ANGABEN ZU SEQ ID NO: 9:
   (i) SEQUENZKENNZEICHEN:
      (A) LÄNGE: 684 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 9:
   CTATTAGTAA AAACAACAGA AAGATAGATT GTATTAGTAA AAACAACAGA AAGATAGAGA 60
   TATGGAGCAA TTATTTTTCT TTTCCTTATT TTAGTTTACA TAATATATAT TATAGGAAGT 120
   TATATATAGA ACGAGAGAGA GTGCTTTGCA TATCCCTATT TATATGTGTA TGTCTTATTT 180
   TGTATTGTTG TTCAGCTTTT ATTTAACTAC ATATTAGCGA ACTCTATTTC TTGTTACTCA 240
   TGTGTTTGTA ATTACATAGC CATGAGGTTT ACGTTTCGAC TATATCGATA TGGAATCGCA 300
   ACTATAATGG TATATAACGA TATATGTAAA TCAGTTATAA AACGACATGT ATAAGGTACG 360
   TATATAAAGT TATATGAATA ACTCAGTTAA TGATATAGAT GCTATAAGTG TATGTGTCTT 420
   AAAACTTCAT TTAGTTTACA TAATCTAAAA TACACTATTA AAAACACACC AGATTCTCAT 480
   ATATCCGGTG TGTTTGCAGT ATTATTCTTG ATAAACATCT ATTAAGGCGT TGGGATAATC 540
   ATCACGGTCT GCACTAAATA TATCTTTTGT TTTGAAATCA TCCACAATTG TACCATTATT 600
   TAACACAAAT CATACGANCA ACAATTTGAT TTAAAGTACT GCAAATATCA TGCGAGGGGG 660
   TATCATCGTA GTTGCTGGTC TTTA 684
(2) ANGABEN ZU SEQ ID NO: 10:
   (i) SEQUENZKENNZEICHEN:
      (A) LÄNGE: 915 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 10:
   CCCTTATAGC AAAAGGAGCT TGAAACTTTG TCATGCTTTT ACTACTTGTT TCCATTGTTC     60
   ATATCACTCA AGTTTCCAAA TCCTCTGCAA ATACGCGTAA TCTTTTTATT GGATTTCGCA    120
   GCCATCGCAC CTATCAAAAT ATCAGCTGGA ATGACATTCA ACACTACTAC CAATCACTTG    180
   AATTAAAATA CTTCAAGCGT CTTTTATATA TCAGCATTTT GTTCGCATTG ATAGACATCA    240
   TTACGTTGCT GAAGTACCAT ACTGCTTGGT TAATCCTTGC AGAATTATGT ATTTACTTCA    300
   TTACACTTAT TTTGTTATCC AAAAAAGTAG AACATTATAT CGCACAAAAA GGAAAGTAGT    360
   TGTAATCCTA TCGAAAGAGG GAGAGTTCGT TCCAAAGTAG GATGGCAGCA GGTATTTAAT    420
   CCGCTATAAT TTAAGTTATT AAATTAGAAA GTCAGGTTTT TAATATGTCA GCAATCCACA    480
   GAAAATATAT TGCCTCTACT TTAATTATCT TAATAGCTTT AATGGTGCTC GTGAAATCGA    540
   ACCTGATTCT TTTCATAGAT GAGCCGGTAT ATCGCTTAGT GAGATTGCTT CGCATATCCC    600
   ATTTGCCAAT ACCTTCTTGT CCTATTACTC AGATATTTTT TCACCTTGGC ATATGGTTGG    660
   TGTAAAGGTT GTTATTTAAC GAATCTCTTA TTCAAAAATC GTCGTCTCGC TTATATTACA    720
   ACTATTTGGG CAACTTCCAC ATTATTGCTC GGCATTTGGT CTGAAATATT TTATCCACCG    780
   TCCAAGACCC AGTAGATTAT ATTTCAGGTT ATAGGTTCCC CAGCTTGGCA TACCATTGGG    840
   CCAATAGCCG GAGCGGCGGT GCCTTTTAAA TGCGGGTCCG GACGATTTTA TTGGCATAAT    900
   CCTGGTTATT AACGG                                                     915
(2) ANGABEN ZU SEQ ID NO: 11:
   (i) SEQUENZKENNZEICHEN:
      (A) LÄNGE: 5 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (iii) HYPOTHETISCH: NEIN
   (xi) SEQUENZBESCHREIBUNG: SEQ ID NO: 11:
   Leu Pro Xaa Thr Gly
   1               5
(2) ANGABEN ZU SEQ ID NO: 12:
   (i) SEQUENZKENNZEICHEN:
      (A) LÄNGE: 35 Basenpaare
      (B) ART: Nucleotid
      (C) STRANGFORM: nicht bekannt
      (D) TOPOLOGIE: linear
   (ii) ART DES MOLEKÜLS: Sonstige Nucleinsäure
   (iii) HYPOTHETISCH: NEIN
   (xi) SEQUENZBESCHREIBUNG: SEQ ID NO: 12:
   ATAAGGCGCC TTAGTTTAAT TATGCTTTGT GATTC          35
(2) ANGABEN ZU SEQ ID NO: 13:
   (i) SEQUENZKENNZEICHEN:
      (A) LÄNGE: 56 Basenpaare
      (B) ART: Nucleotid
      (C) STRANGFORM: nicht bekannt
      (D) TOPOLOGIE: linear
   (ii) ART DES MOLEKÜLS: Sonstige Nucleinsäure
   (iii) HYPOTHETISCH: NEIN
   (xi) SEQUENZBESCHREIBUNG: SEQ ID NO: 13:
   CGCAGGAAGC TTACCACAAT CTAAGAAATC TGAAATATCT CAAGCAAGTG GAGAAG          56
(2) ANGABEN ZU SEQ ID NO: 14:
   (i) SEQUENZKENNZEICHEN:
      (A) LÄNGE: 38 Basenpaare
      (B) ART: Nucleotid
      (C) STRANGFORM: nicht bekannt
      (D) TOPOLOGIE: linear
   (ii) ART DES MOLEKÜLS: Sonstige Nucleinsäure
   (iii) HYPOTHETISCH: NEIN
   (xi) SEQUENZBESCHREIBUNG: SEQ ID NO: 14:
   AATAAGGCGC CTCATTATCC ACCTGTTTCA GGTAGTTC 38
(2) ANGABEN ZU SEQ ID NO: 15:
   (i) SEQUENZKENNZEICHEN:
      (A) LÄNGE: 33 Basenpaare
      (B) ART: Nucleotid
      (C) STRANGFORM: nicht bekannt
      (D) TOPOLOGIE: linear
   (ii) ART DES MOLEKÜLS: Sonstige Nucleinsäure
   (iii) HYPOTHETISCH: NEIN
   (xi) SEQUENZBESCHREIBUNG: SEQ ID NO: 15:
   ACGAAAGCTT ACCACAATCT AAGAAATCTG AAC 33

## Beschreibung

Gegenstand der vorliegenden Erfindung ist ein Verfahren zur Bestimmung von Wirksubstanzen, welche die kovalente Verknüpfung von Polypeptiden an die Oberfläche von Gram-positiven Bakterien beeinflussen.

Angesichts des vermehrten Auftretens antibiotikaresistenter Stämme stellen durch Gram-positive Bakterien hervorgerufene Infektionen beim Menschen eine zunehmende therapeutische Herausforderung dar. Eine Vielzahl von bakteriellen Oberflächenproteinen ist mit der Pathogenese dieser Organismen verbunden. So sind zellwandverankerte Pathogenitätsfaktoren bekannt, welche die bakterielle Adhäsion durch Bindung an extrazelluläre Matrixkomponenten der Wirtsgewebe, wie z.B. Kollagen, fördern. Andere Faktoren binden Serumkomponenten wie IgG und verbergen somit die authentische Bakterienoberfläche vor dem Immunsystem des Wirtes. Eine gezielte Hemmung der Verknüpfungsreaktion dieser Proteine an die bakterielle Zellwand ist daher von großem medizinischen Interesse.

Schneewind et al. (Cell, Vol. 70, p. 267 - 281, 1992) untersuchen den Verankerungsmechanismus von Protein A in der Zellwand von Staphylokokken. Protein A gehört zu einer wachsenden Klasse von Oberflächenproteinen Gram-positiver Bakterien, welche durch eine Abfolge des charakteristischen Sequenzmotives·LPXTG, gefolgt von einer Gruppe aus 15 - 22 hydrophoben Aminosäuren sowie einer am C-terminalen Ende befindlichen Gruppe aus 5 - 12 geladenen Aminosäuren gekennzeichnet sind. Die Konservierung dieser Elemente wird als Indikation eines gemeinsamen Ausschleusungsmechanismus dieser Proteine in unterschiedlichen Gram-positiven Spezies angesehen. Um die Lokalisation des Protein A (Unterscheidung zwischen in der Zellwand verankertem und sezerniertem Protein A) in *S. aureus* feststellen zu können, greifen die Autoren auf radioaktive Labellingverfahren zurück. Die Bedeutung der oben genannten Sequenzelemente für die Zellwandverankerung wird mit Hilfe von Hybridproteinen sowie durch Mutagenese des LPXTG-Motives und des C-Terminus untermauert. Schneewind et al. befassen sich jedoch weder mit möglicherweise die Verankerung der Oberflächenproteine katalysierenden Enzymen noch mit deren Hemmung.

Die Zellwandverankerungselemente in Oberflächenproteinen Gram-positiver Bakterien sind ebenfalls Thema eines weiteren Artikels von Schneewind et al. (EMBO J., Vol. 12, p. 4803 - 4811, 1993). Es wird gezeigt, daß Enterotoxin B, ein normalerweise in das Medium sezerniertes Protein, mittels C-terminaler Fusion an das Protein A-Verankerungssignal in der Staphylokokken-Zellwand verankert werden kann. Die Ergebnisse unterstützen die Hypothese, daß das Zellwand-Sorting mit einer proteolytischen Spaltung der Polypeptidkette am C-Terminus einhergeht. Vermutlich ist das LPXTG-Motiv die Stelle dieser Spaltung und kovalenter Zellwandverknüpfung, während der geladene Sequenzabschnitt als Retentionssignal während des Zellwand-Sortings dient. Die Relevanz der faltungsbedingten geometrischen Länge der hydrophoben Domäne wird experimentell bestätigt.

Mit dem Zelloberflächendisplay rekombinanter Proteine auf *Staphylococcus carnosus* befaßt sich ein Artikel von Samuelson et al. (J. Bacteriol., Vol. 177, no. 6, p. 1470 - 1476, 1995). Der Oberflächendisplay des Malariapeptides M3 erfolgt hier unter Verwendung von Promoter, Sekretionssignal und Propeptidregion des Lipasegens von *S*. *hyicus* sowie der Zellwandverankerungsregionen des Protein A von *S*. aureus. Die hybride Proteinstruktur beinhaltet ferner ein Serumalbuminbindeprotein, welches der Detektion der rekombinanten, oberflächen-verankerten Proteine in einem colorimetrischen Sandwichassay dient. Weitere Detektionsmethoden umfassen die Immunogold-Elektronenmikroskopie, Immunofluoreszenzassays sowie das fluoreszenzaktivierte Cellsorting (FACS). Auch Samuelson et al. befassen sich nicht mit den genauen molekularen Mechanismen der Zellwandverankerung.

Die Struktur des Zellwandankers der Oberflächenproteine in *Staphylococcus aureus* ist Thema eines Reports von Schneewind et al. (Science, Vol. 268, p. 103 - 106, 1995). Die Autoren verwenden eine Kombination molekularbiologischer und massenspektrometrischer Techniken und können zeigen, daß nach Spaltung des Oberflächenproteins zwischen Threonin und Glycin des konservierten LPXTG-Motives die Carboxylgruppe des Threonins durch Transpeptidierung mit der freien Aminogruppe des Zellwand-Pentaglycins kovalent an den Mureinsacculus gebunden wird. Das für die Proteolyse und Transpeptidierung vermutlich verantwortliche Protein, die sogenannte Sortase, wird jedoch auch von Schneewind et al. nicht identifiziert bzw. charakterisiert.

Strauß und Götz (Molecular Microbiology, Vol. 21, p. 491 - 500, 1996) befassen sich mit der *in vivo* Immobilisierung enzymatisch aktiver Polypeptide auf der Zelloberfläche von *Staphylococcus carnosus.* Sie konstruieren ein Hybridprotein, welches aus *Staphylococcus hyicus* Lipase und der C-terminalen Region des *Staphylococcus aureus* Fibronectin Bindeprotein B (FnBPB) besteht. Um die Zellwandassoziation der Prolipase bzw. des pro-LipFnBPB-Hybrides zu untersuchen, verwenden die Autoren ein prolipasespezifisches Antiserum in einem Immunofluoreszenzassay sowie Immunoblotting. Weitere Untersuchungen belegen, daß für eine effiziente Faltung der Lipase in ihre aktive Konformation offenbar ein Abstand von ca. 90 Aminosäuren zwischen dem C-Terminus des Enzyms und dem Zellwand-Sortingsignal unerläßlich ist. Der Einfluß größerer Abstände wurde an Fusionen aus proLip und der C-terminalen Region von *S*. *aureus* Protein A (proLipSPA, Spacer mit 165 Aminosäuren) und *S*. *aureus* Fibronectin Bindeprotein A (proLipFnBPA, Spacer mit 223 Aminosäuren) untersucht. Zusätzliche Experimente wurden mit *E. coli β*-Lactamase als Reportermolekül durchgeführt.

Die WO-A-97/08553 beschreibt ein Verfahren zum stabilen, nicht-kovalenten Display von Proteinen, Peptiden und anderen Substanzen auf der Oberfläche von Gram-positiven Bakterien. Es wurden vergleichende Untersuchungen zwischen dem nicht-kovalenten sowie den oben näher beschriebenen kovalenten Displayverfahren durchgeführt. Bei Ersatz des C-terminalen Sorting-Signals von Protein A, welches ein kovalentes Display generiert, durch das Zellwand Targeting-Signal von Lysostaphin (SPA_{CWT}) konnte eine im wesentlichen unveränderte Bindungsintensität von FITC-markiertem IgG an die Staphylokokken-Oberfläche beobachtet werden.

Die US-A-5,616,686 offenbart ein aus ca. 6 bis 20 Aminosäuren bestehendes Polypeptid, welches als integralen Bestandteil ein für die Verankerung von virulenzdeterminierenden Proteinen auf der Oberfläche von Gram-positiven Bakterien verantwortliches Peptidkonstrukt enthält. Insbesondere ist dieses Konstrukt dadurch gekennzeichnet, daß es an den Positionen 1, 2, 4 und 5 der Aminosäuresequenz die Aminosäuren L, P, T und G aufweist. Aufgrund der Homologie dieser Peptide mit den Sequenzen der Virulenzdeterminanten in den Wildtyp-Oberflächenproteinen reagieren erstere vermutlich mit den an der Verankerung beteiligten Enzymen. Hieraus resultiert, daß die Virulenzdeterminanten der Bakterien nicht oder nur in geringerem Maße verankert werden können und so ein Fortschreiten der Infektion unterbunden wird. Das bzw. die an der Oberflächenverankerung beteiligte(n) Enzym bzw. Enzyme werden jedoch auch in dieser Patentschrift nicht charakterisiert.

Die WO-A-93/18163 befaßt sich mit der Bereitstellung von Fusionsproteinen, welche mindestens die Ankerregion Gram-positiver Oberflächenproteine sowie variable Proteine, Polypeptide oder Peptide mit insbesondere therapeutischer Wirkung in Mensch und Tier enthalten. Die Ankerregion umfaßt ein LPSTGE-Segment, ein Spacer-Segment der Sequenz TAN, ein aus 20 Aminosäuren bestehendes hydrophobes Segment sowie einen geladenen Segmentabschnitt mit der Sequenz KRKEEN.

Es ist wünschenswert, ein Verfahren bereitzustellen, welches die Bestimmung von Wirksubstanzen erlaubt, die die kovalente Verknüpfung von Polypeptiden an die Oberfläche von Gram-positiven Bakterien direkt oder indirekt beeinflussen.

Überraschenderweise wird dieses Ziel durch das Verfahren der vorliegenden Erfindung erreicht.

Polypeptide im Sinne der Erfindung bezeichnet aus in der Regel mindestens 20 Aminosäuren aufgebaute Polymere und umfaßt insbesondere auch Proteine. Die Aminosäuren sind durch den 1-Buchstaben-Code dargestellt, wobei X für eine beliebige Aminosäure steht.

Zunächst soll im folgenden die Grundlage einer bevorzugten Ausführungsform des erfindungsgemäßen Verfahrens dargestellt werden, bevor im einzelnen auf das Verfahren zur Identifizierung von Wirksubstanzen eingegangen wird.

Das erfindungsgemäße Verfahren ist in einer bevorzugten Ausführungsform als enzymatischer Reporterassay aufzufassen, der die Wirkung von Substanzen auf bakterielle Faktoren (Targets) erfaßt, welche direkt oder indirekt an der von einem LPXTG-Motiv abhängigen C-terminalen Verankerung von Polypeptiden an der Oberfläche von Gram-positiven Bakterien mitwirken. Aus der Vielzahl von Faktoren und Vorgängen, die sich auf diesen Prozess auswirken können, zielt das vorliegende Verfahren vorzugsweise auf solche enzymatischen Schritte, die nach dem Beginn der Translokation der Zelloberflächen-Polypeptide über die Cytoplasmamembran stattfinden. Darüber hinaus erfaßt das erfindungsgemäße Verfahren zum Teil enzymatische und andere Targets, die an der Biosynthese des Zellwandmureins mitwirken. Anhand der phänotypischen Merkmale der im jeweiligen Verfahren verwendeten Zellen lassen sich potentielle Wirkstoffe bestimmten Targetgruppen zuordnen.

In einer besonders bevorzugten Ausführungsform des erfindungsgemäßen Verfahrens bildet die zelluläre und molekulare Grundlage des Reporterassays ein rekombinanter *Staphylococcus carnosus* Klon, welcher ein selektierbares Expressionsplasmid mit einer induzierbaren Reportergenfusion enthält. Die Genfusion kodiert für ein Hybridpolypeptid bestehend aus einem N-terminalen Signalpeptid, einem Vorläuferprotein der Lipase aus *Staphylococcus hyicus* und einem C-terminalen Anteil des Fibronektin-Bindeproteins B (FnBPB) aus *Staphylococcus aureus*. Nach seiner Produktion im Cytoplasma wird das Hybridpolypeptid aufgrund seiner N-terminalen Signalstrukturen durch die bakterielle Zellmembran transportiert und am Aminoende durch eine Signalpeptidase prozessiert. Ferner erfolgt eine Spaltung im C-terminalen LPXTG-Erkennungsmotiv und das verbleibende Hybridprotein wird kovalent mit dem Murein verknüpft. Auf experimentellem Wege wurde ermittelt, daß unterschiedliche Längen des FnBPB-Anteils die Ausbildung enzymatischer Aktivität der Lipasefusionen in unterschiedlicher Weise beeinflussen. Ein Konstrukt wurde identifiziert, das in seiner Zelloberflächen-gebundenen Form keine Lipaseaktivität aufwies (kodiert von Plasmid pTX30Δ82). Wurde die entsprechende Fusion jedoch nach ihrer kovalenten Verankerung durch Lysostaphinbehandlung von der Bakterienoberfläche freigesetzt, wurde die volle Lipaseaktivität erzielt. Im Rahmen der vorliegenden Erfindung wurde erstmals erkannt, daß in dem betreffenden Assayklon Störungen in der Zellwandverankerung der Lipasefusion zur Freisetzung der Fusion verbunden mit dem Auftreten von Lipaseaktivität in den Kulturüberständen führen. Als mögliche Angriffspunkte kommen verschiedene zelluläre Faktoren in Betracht, die entsprechend ihrem Wachstumsverhalten im wesentlichen in zwei Gruppen eingeteilt werden können.

Ein Target bzw. eine Targetgruppe ist das als Sortase bezeichnete Enzym bzw. der Enzymkomplex, das bzw. der an der Oberfläche der Bakterien die carboxyterminale Spaltung relevanter Polypeptide im LPXTG-Motiv und deren anschließende kovalente Verknüpfung an Peptidbestandteile des Zellwandmureins wie z.B. Interpeptidbrücken, insbesondere Pentaglycineinheiten, bewerkstelligt. Die Inhibierung dieser Funktionen führt zwar vermutlich zur Freisetzung oberflächengebundener Faktoren und damit wahrscheinlich zur Attenuierung pathogener Bakterien, wohl aber nicht zu maßgeblichen Beeinträchtigungen in der Lebens- und Teilungsfähigkeit der Bakterien. Kennzeichnendes phänotypisches Merkmal dieser Targetgruppe im Assayverfahren ist die Freisetzung von Lipaseaktivität in das Assaymedium bei mehr oder weniger unbeeinträchtigtem Wachstumsverhalten der Bakterien. Im Gegensatz dazu führt die Beeinträchtigung eines Targets bzw. einer Targetgruppe mit essentieller Funktion in der Mureinsynthese zu massiven phänotypisch erfassbaren Veränderungen in der Lebens- und Teilungsfähigkeit der Bakterien.

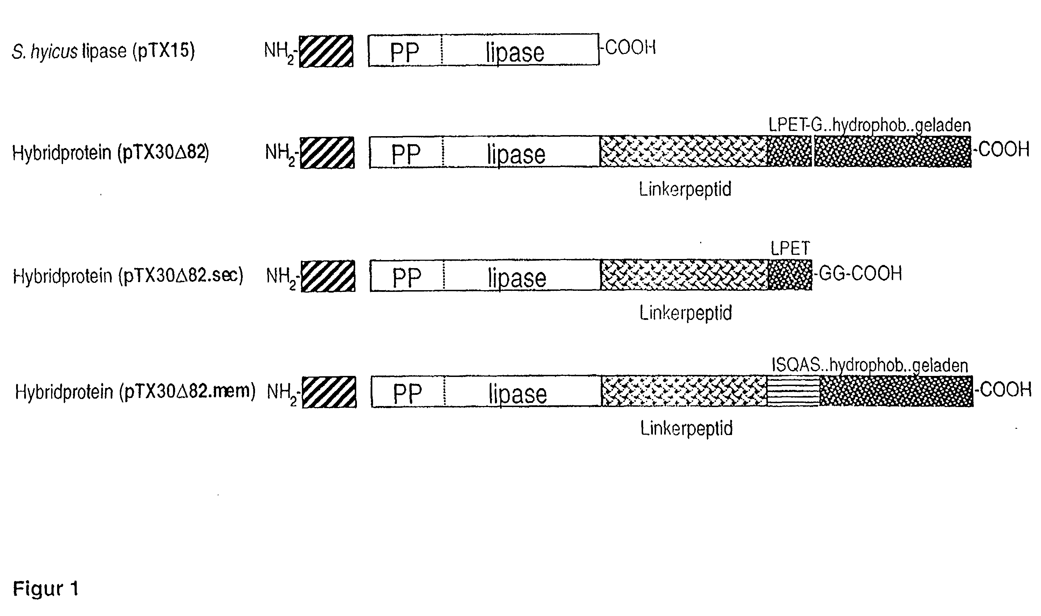

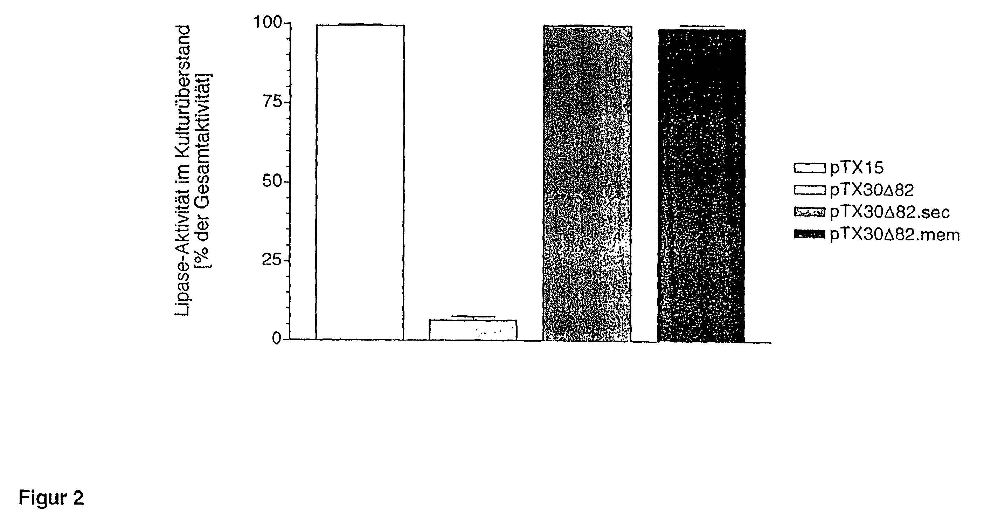

Um die potentielle Eignung des erfindungsgemäßen Verfahrens für die Untersuchung des Targets "Sortase" zu demonstrieren, wurden zwei unterschiedliche Hemm-/Mutations-Szenarien mit Hilfe gentechnisch gezielt veränderter Hybridproteine simuliert (siehe hierzu Ausführungsbeispiel 1 sowie Figuren 1 und 2).

*S*. *carnosus/* pTX30Δ82.mem produziert nach Induktion des Xylose-Promoters ein Hybridprotein bestehend aus der *S*. *hyicus* Prolipase und dem C-terminalen Fragment des *S*. *aureus* Fibronectin-Bindeprotein B (FnBPB), wobei das für die Verankerung mit der Zellwand wichtige LPXTG-Motiv gegen die Sequenz ISQAS ausgetauscht wurde (Figur 1). In anderen Ausführungsformen kann jedoch auch ein Austausch gegen eine beliebige andere, bevorzugt aus 5 Aminosäuren bestehende Sequenz erfolgen. In einer weiteren Ausführungsform ist für den mem-Phänotyp eine LPXTG-Substitution nicht notwendig, sondern auch eine komplette Deletion des LPXTG-Motivs denkbar. Damit entfällt die spezifische Spaltung am LPXTG-Motiv und somit die kovalente Verknüpfung an den Mureinsacculus. Überraschenderweise konnte gezeigt werden, daß die hydrophobe Ankersequenz, die somit am Carboxylende der Fusion erhalten bleibt, offenbar nicht ausreichend ist, um die Lipase stabil und insbesondere in inaktiver Form in der Zellhülle und somit an der Zelloberfläche zu verankern. Es kommt zur quantitativen Freisetzung der Lipaseaktivität von der Bakterienoberfläche in das Kulturmedium. Dieser Klon simuliert somit die Inhibition der Spaltungsreaktion durch die Sortase, die als wesentlicher Schritt der kovalenten Verknüpfung des N-terminalen Spaltprodukts an das Murein der Zellwand vorangeht.

*S. carnosus/* pTX30Δ82.sec produziert nach Induktion des Xylose-Promoters ein Hybridprotein bestehend aus der *S*. *hyicus* Prolipase und einem C-terminalen Fragment des *S*. *aureus* Fibronectin-Bindeprotein B (FnBPB), welches mit dem Motiv LPETGG endet (Figur 1). Dieser Klon simuliert die Inhibition der (kovalenten) Verknüpfungsreaktion zwischen dem zu verankernden, C-terminal bereits prozessierten Lipase-Hybridprotein und der Zellwand. Das Lipase-Hybridprotein wird von diesem Klon quantitativ in aktiver Konformation in den Kulturüberstand freigesetzt.

Die Bestimmung von Wirksubstanzen, welche die kovalente Verknüpfung von Polypeptiden an die Oberfläche von Gram-positiven Bakterien beeinflussen, erfolgt erfindungsgemäß durch ein Verfahren mit den folgenden Schritten:
a) Bereitstellen einer Probe Gram-positiver Bakterien, welche mindestens eine kovalent mit der Oberfläche der Gram-positiven Bakterien verbundene oder verbindbare enzymatische Reportersubstanz aufweisen oder bilden, wobei die mindestens eine Reportersubstanz ohne kovalente Bindung an die Oberfläche der Gram-positiven Bakterien eine andere enzymatische Aktivität aufweist als bei kovalenter Bindung an die Oberfläche der Gram-positiven Bakterien,
b) Inkontaktbringen der Probe mit einer möglichen Wirksubstanz,
c) Bestimmen der enzymatischen Aktivität der Reportersubstanz der Gram-positiven Bakterien der Probe.

Im Sinne des erfindungsgemäßen Verfahrens kann "ohne kovalente Bindung" sowohl nicht-kovalente Bindung an die Oberfläche der Gram-positiven Bakterien als auch vollständige Freisetzung bedeuten.

Die Bestimmung der enzymatischen Aktivität der Reportersubstanz der Gram-positiven Bakterien der Probe kann in bevorzugter Weise im Vergleich zu mindestens einer Referenzprobe, die nicht mit der Wirksubstanz in Kontakt gebracht wurde, und/oder mindestens einer Referenzprobe, in der die Reportersubstanz nicht-kovalent an die Oberfläche Gram-positiver Bakterien gebunden ist, und/ oder mindestens einer Referenzprobe, in der die Reportersubstanz kovalent an die Oberfläche der Gram-positiven Bakterien gebunden ist, und/oder mindestens einer Referenzprobe, in der die Reportersubstanz ohne kovalente Bindung an die Oberfläche der Gram-positiven Bakterien vorliegt, erfolgen.

Das erfindungsgemäße Verfahren erlaubt in vorteilhafter Weise selektiv die Identifizierung von solchen Wirksubstanzen, welche die kovalente Verknüpfung von Polypeptiden an die Oberfläche von Gram-positiven Bakterien direkt oder indirekt beeinflussen. Wie eingangs erläutert, umfaßt der putative Prozeß der Oberflächenverankerung zwei spezifische Schritte:
a) Spaltung zwischen Threonin und Glycin des LPXTG-Motives und
b) kovalente Verknüpfung des Threonins an Peptidbestandteile, insbesondere an die Interpeptidbrücke, der Zellwand.

Mittels des erfindungsgemäßen Verfahrens werden somit sowohl solche Wirksubstanzen erfaßt, die die Spaltungsreaktion inhibieren und somit auch eine kovalente Verknüpfung der Polypeptide unterbinden als auch solche Wirksubstanzen, die eine inhibitorische Funktion im zweiten Schritt der Oberflächenverankerung besitzen. Diese Szenarien wurden, wie eingangs dargestellt, mit Hilfe von gentechnisch gezielt veränderten Reportersubstanzen simuliert und somit die Grundlage für das erfindungsgemäße Verfahren geschaffen. Das erfindungsgemäße Verfahren erfaßt zudem auch solche Wirksubstanzen, die z.B. durch Hemmung von Zellwandbiosynthese-Enzymen einen inhibierenden Einfluß auf die Zellwandbiosynthese haben, so daß als Folge eine kovalente Verknüpfung von Polypeptiden, insbesondere Pathogenitätsfaktoren, an die Zellwand, hier insbesondere an Interpeptidbrücken des Mureinsacculus, nicht oder nur noch eingeschränkt möglich ist. Darüber hinaus werden durch das erfindungsgemäße Verfahren auch Wirksubstanzen erfaßt, die z.B. durch Aktivierung von Zellwand-Hydrolasen, bereits kovalent in der Zellwand verankerte Polypeptide freisetzen.

In einer bevorzugten Ausführungsform des erfindungsgemäßen Verfahrens wird als Reportersubstanz ein Hybridpolypeptid verwendet, welches insbesondere eine Anordnung der folgenden Sequenzabschnitte aufweist: N-terminales Signalpeptid, Enzym, Sequenzabschnitt mit der Sequenz LPXTG, hydrophober Sequenzabschnitt und geladener Sequenzabschnitt. Das Signalpeptid wird im Rahmen des sekretorischen Prozessierungsweges proteolytisch entfernt. Wie in den nachfolgenden Beispielen gezeigt, kann insbesondere als enzymatischer Bestandteil des Hybridpolypeptides *S*. *hyicus* Lipase verwendet werden. Es kann jedoch auch von Vorteil sein, *E*. *coli* b-Lactamase oder andere Enzyme zu verwenden. Natürlich vorkommende Oberflächenpolypeptide können ebenso wie gentechnisch hergestellte Hybridpolypeptide als Reportersubstanzen dienen, die infolge der Wirkung geeigneter Wirksubstanzen im Medium mit Hilfe der gesamten Palette der bekannten chemischen, biochemischen und immunologischen Methoden nachgewiesen werden können. Um allerdings einen hohen Probendurchsatz bei einer ungeminderten Verläßlichkeit des erfindungsgemäßen Verfahrens zu gewährleisten, ist es insbesondere vorteilhaft, als Reportersubstanzen Hybridpolypeptide mit enzymatischer Aktivität zu verwenden. Wirksubstanzen, welche die proteolytische Spaltung des Signalpeptides wie auch den gesamten Transportweg durch die cytoplasmatische Membran beeinflussen, führen somit nicht zum Auftreten der aktiven Reportersubstanz im Überstand.

Es kann darüber hinaus bevorzugt sein, Reportersubstanzen mit mindestens einer detektierbaren Eigenschaft zu verwenden, wobei die Reportersubstanz ohne kovalente Bindung an die Oberfläche der Gram-positiven Bakterien eine veränderte detektierbare Eigenschaft aufweist als bei kovalenter Bindung an die Oberfläche der Gram-positiven Bakterien.

Es kann weiterhin bevorzugt sein, das Enzym als Proenzym bereitzustellen.

Darüber hinaus kann es insbesondere bevorzugt sein, die Änderung der enzymatischen Aktivität durch den Übergang des Enzyms aus einer inaktiven in eine aktive Konformation oder umgekehrt, zu bestimmen. Dies kann bevorzugt durch Verwendung eines zwischen Enzym und LPXTG-Motiv angeordnetem Linkerpeptid erzielt werden.

In einer bevorzugten Ausführungsform des erfindungsgemäßen Verfahrens ist die Anzahl der Aminosäuren des Linkerpeptides so gewählt, daß das Enzym in einer inaktiven Konformation an der Oberfläche der Gram-positiven Bakterien verankert wird. Bei Verwendung von *S*. *hyicus* Lipase sollte die Anzahl der Aminosäuren im Linkerpeptid insbesondere weniger als zehn betragen. In einer weiteren Ausführungsform könnte unter Verzicht auf das Linkerpeptid das Enzym mit seinem C-Terminus direkt an das LPXTG-Motiv fusioniert vorliegen. In Abwesenheit der Wirksubstanz tragen die Gram-positiven Bakterien somit kovalent an ihrer Oberfläche gebundene inaktive Enzyme, welche bei nichtkovalenter Verknüpfung, insbesondere bei Freisetzung von der Oberfläche der Gram-positiven Bakterien, in eine aktive Konformation falten und somit eine bestimmbare Veränderung in einer ihrer Eigenschaften, d.h. in diesem Fall der enzymatischen Aktivität, erfahren.

Es ist insbesondere bevorzugt, das erfindungsgemäße Verfahren mit solchen Gram-positiven Bakterien durchzuführen, die einen geringen natürlichen Zellwand-turn-over und/oder eine geringe Anzahl an Zellwandproteasen und/oder eine geringe Anzahl an sekretierten Proteasen aufweisen, um den Anteil an falschpositiven Ergebnissen bei der Suche nach geeigneten Wirksubstanzen zu minimieren. Somit kann das erfindungsgemäße Verfahren nicht nur mit natürlich vorkommenden Gram-positiven Bakterien wie z.B. *S. carnosus* durchgeführt werden, sondern auch mit bereits genetisch veränderten Bakterien.

Darüber hinaus kann es ebenfalls bevorzugt sein, daß erfindungsgemäße Verfahren an Lif-exprimierenden Zellen durchzuführen. Lysostaphin Immunity Factor (Lif) exprimierende Gram-positive Zellen zeigen Modifikationen im Mureingerüst der Zellwand. Diese Veränderungen haben keinen Einfluß auf die Verknüpfungsreaktionen von Zelloberflächenproteinen in der Zellwand. Das Ausführungsbeispiel 2 beschreibt eine bevorzugte Ausführungsform des erfindungsgemäßen Verfahrens zur Untersuchung der Verankerung von Proteinen in der Zellwand Lifexprimierender Zellen anhand der beispielhaft ausgewählten *S. hyicus* Lipase oder proLipFnBPB. Der Vergleich der enzymatischen Aktivität der Lipase an der Zellwand und im Überstand des Kulturmediums zeigte, daß die Lif-Expression keinen Einfluß auf die Sekretion der Lipase oder die Verankerung von proLipFnBPB in der Zelloberfläche hat.

In einer besonders bevorzugten Ausführungsform des erfindungsgemäßen Verfahrens erfolgt die Bestimmung der enzymatischen Aktivität der Reportersubstanz mittels Fluoreszenzspektroskopie, insbesondere konfokaler Fluoreszenzspektroskopie wie in Ausführungsbeispiel 1 gezeigt. Es ist hierbei möglich, auf die bekannten Verfahren der Ein- oder Mehrphotonenanregung zurückzugreifen. Als vorteilhafte Bestimmungsmethode hat sich insbesondere das Verfahren der Fluoreszenzkorrelationsspektroskopie (FCS) erwiesen, wie es in der WO-A-94/16313 detailliert beschrieben ist. Anstelle der in der vorgenannten Patentanmeldung beschriebenen Vorrichtung kann es auch bevorzugt sein, FCS unter Verwendung von Elementen aus der Nahfeldspektroskopie durchzuführen, wie in DE-C-44 38 391 und der korrespondierenden WO-A-96/13744 dargelegt. Auf diese Literaturzitate wird zur Ergänzung der Beschreibung ausdrücklich Bezug genommen.

Die WO-A-98/16814 beschreibt ein Verfahren zur Analyse von Partikel enthaltenden Proben durch wiederholte Messung der Photonenanzahl pro definiertem Zeitintervall von emittiertem oder in der Probe gestreutem Licht, gefolgt von der Bestimmung der Verteilungsfunktion der Photonenanzahl pro definiertem Zeitintervall, aus welcher sodann die Verteilungsfunktion der Partikelhelligkeiten bestimmt wird. Dieses Verfahren kann in bevorzugter Weise auch zur Untersuchung lumineszenter, insbesondere fluoreszierender, Proben eingesetzt werden und wird in dieser spezifischen Ausführungsform als sogenannte Fluoreszenzintensitätsverteilungsanalyse (fluorescence intensity distribution analysis, FIDA) bezeichnet. Auf den Offenbarungsgehalt dieser Literaturstellen wird zur Ergänzung der Beschreibung ausdrücklich Bezug genommen.

Für Fluoreszenzmessungen geeignete Farbstoffe sind dem Fachmann aus der Literatur bekannt. Es kann z.B. bevorzugt sein, die Umsetzung eines Substrates, welches eine Änderung in seinen Fluoreszenzeigenschaften erfährt, zu bestimmen. Weiterhin kann es bevorzugt sein, einen Reporterassay unter Verwendung von fluoreszierenden bzw. luminogenen Proteinen wie beispielsweise GFP (Green Fluorescent Protein) einzusetzen.

In einer weiteren Ausführungsform des erfindungsgemäßen Verfahrens ist es möglich, Gruppen von möglichen Wirksubstanzen der Probe Gram-positiver Bakterien zuzugeben, um bei positivem Signal eine weitere Differenzierung dieser Gruppe vorzunehmen. Desweiteren kann der Probe, falls eine mögliche Wirksubstanz nicht positiv mit der Probe interferiert, eine weitere mögliche Wirksubstanz zugegeben werden, ohne die Probe zu wechseln. In diesem Fall werden sequentiell mögliche Wirksubstanzen zugegeben. Es ist in diesem Fall auch möglich, synergistische Effekte festzustellen, wenn sich die als wirksam erwiesene Substanz in einem Bestätigungsversuch anders verhält als im Gemisch mit der oder den scheinbar nicht wirksamen Substanzen.

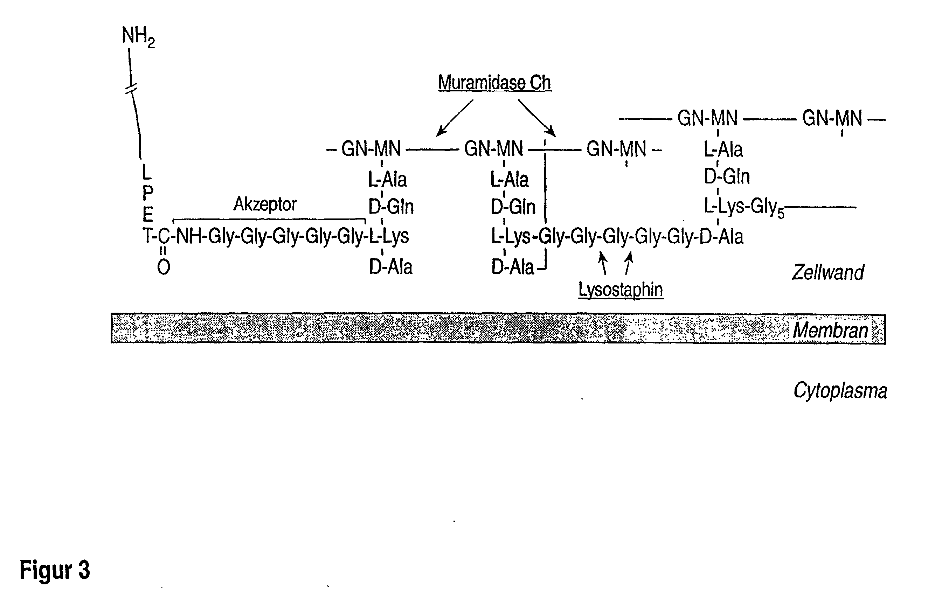

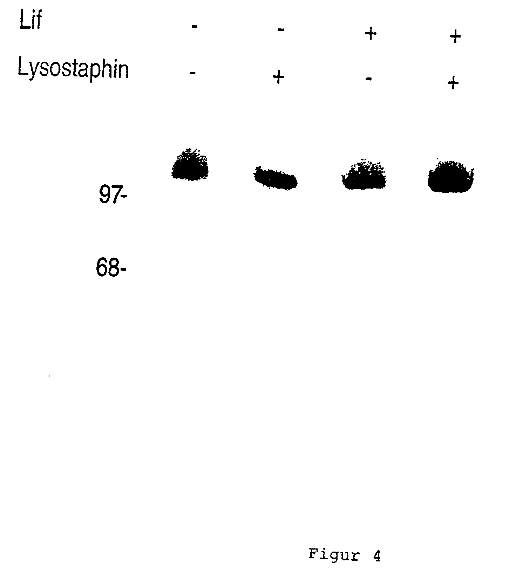

Eine weitere Ausführungsform des erfindungsgemäßen Verfahrens erfaßt neben der Bestimmung der enzymatischen Aktivität der Reportersubstanzen deren Verankerung an der Zelloberfläche. Diese im Ausführungsbeispiel 3 beschriebene weitere Form des erfindungsgemäßen Verfahrens ermöglicht es festzustellen, ob Proteine durch nicht-kovalente Bindungen an einer Freisetzung gehindert werden. Diese besondere Ausführungsform erlaubt es, zu bestimmen, auf welche Weise die Reportersubstanz an das Mureingerüst gebunden ist. Muramidase Ch schneidet vorzugsweise gemäß Figur 3 die Zellwand Gram-positiver Bakterien, so daß in der Zellwand verankerte Proteine mit Zellwandfragmenten variabler Länge abgespalten werden (Schneewind et al., EMBO J. 12: 4803-4811, 1993). Nicht-kovalent gebundene Proteine hingegen, die durch Muramidase Ch abgespalten werden, haben alle dieselbe Molmasse (Schneewind et al., EMBO J. 12: 4803-4811, 1993). Auf diese Literaturzitate wird zur Ergänzung der Beschreibung ausdrücklich Bezug genommen. Die Unterscheidung der verschiedenen Spaltprodukte erfolgt gemäß der besonderen Ausführungsform durch SDS-PAGE (Sodiumdodecylsulfat-Polyacrylamid-gelelektrophorese) und Immunoblotting. Beispielhaft ist das SDS-PAGE Laufverhalten von kovalent verankerten Proteinen nach Freisetzung durch Muramidase Ch und Lysostaphin-Behandlung in Figur 4 gezeigt.

In einer besonders bevorzugten Form des erfindungsgemäßen Verfahrens kann im Kulturmedium unterschieden werden, ob die Freisetzung von Zellwandproteinen durch Wirkung von Substanzen, die den Mechanismus der Verankerung an der Oberfläche von Gram-positiven Bakterien beeinflussen oder durch natürliche Änderungen der Zellwand verursacht wurde. Zusätzlich zur erfindungsgemäßen Bestimmung der enzymatischen Aktivität erfolgt eine Charakterisierung der freigesetzten Polypeptide. Ausführungsbeispiel 4 zeigt dieses am Beispiel von proLipFnBPB aus pTX30 und pTX30/pCXlif exprimierenden Zellen. ProLipFnBPB, das durch natürliche Änderungen der Zellwand aus diesen Zellen freigesetzt wurde, zeigt bei der gelelektrophoretischen Untersuchung und anschließendem Immunoblotting einen Schmier aus Lipasen unterschiedlicher Länge. Direkt aus der Zelle freigesetzte Zellwandproteine zeigen, wie die durch Einwirkung von Lysostaphin (80 mg ml⁻¹ in BM, 30 min 37°C) gewonnenen Proteine aus dem Überstand von nicht-Lif exprimierenden Zellen, statt einer verschmierten aus einer Summe aus lipasespezifischen Signalen bestehenden Bande, eine schmale, stark begrenzte Bande bei der SDS-PAGE und anschließendem Immunoblotting.

Die Figur 1 beschreibt beispielhaft die Struktur eines als Reportersubstanz einsetzbaren Hybridproteins (Assay-Plasmid pTX30Δ82) sowie die oben erwähnten Strukturen der zur Simulation der erfindungsgemäßen Verfahren verwendeten Hybridproteine (Plasmide pTX30Δ82.sec und pTX30Δ82.mem) im Vergleich zur Struktur der *S*. *hyicus* Lipase (Plasmid pTX15). Am N-Terminus befindet sich das sogenannte Signalpeptid (schraffiert dargestellt), welches den Transport des Proenzyms (schematisch durch PP und Lipase dargestellt) durch die cytoplasmatische Membran ermöglicht und im Rahmen des sekretorischen Prozessierungsprozesses proteolytisch entfernt wird. In bevorzugter Weise schließt sich an die Lipase ein Linkerpeptid an, dessen Länge derart gewählt ist, daß die Lipase in einer inaktiven Konformation an der Oberfläche der Gram-positiven Bakterien kovalent verankert werden kann. Es folgen LPETG-Motiv, hydrophober und geladener Sequenzabschnitte (Assay-Plasmid pTX30Δ82). Das LPXTG-Motiv ist im zur Simulation verwendeten Hybridprotein (Plasmid pTX30Δ82.mem) durch die Sequenzabfolge ISQAS ersetzt. In dem zu einer weiteren Simulation verwendeten Hybridprotein (pTX30Δ82.sec) schließt sich an das Linkerpeptid lediglich ein Sequenzabschnitt mit der Sequenz LPETGG an.

Figur 2 zeigt die Quantifizierung der Lipaseaktivität in den Simulations-Klonen. Die jeweilige Lipaseaktivität im Kulturüberstand wurde in Relation zur Gesamtaktivität des entsprechenden Klons bestimmt.

Figur 3 zeigt die Struktur des Peptidoglycans in Staphylococcen mit einem C-terminal verknüpften Oberflächenprotein. Die Schnittstellen für Muramidase Ch und Lysostaphin sind hervorgehoben.

In Figur 4 ist der Einfluß von Lysostaphin auf Zelloberflächenproteine, die durch Muramidase Ch aus der Zellwand von *S. carnosus* freigesetzt wurden, dargestellt. ProLipFnBPB wurde in Gegenwart (+) oder Abwesenheit (-) von Lif (pCXLif) in diesen Zellen synthetisiert. Die freigesetzten Hybridproteine wurden in Gegenwart (+) oder Abwesenheit (-) von Lysostaphin inkubiert und anschließend durch SDS-PAGE (10% Acrylamid) und Immunoblotting (Prolipase-spezifisches Antiserum) gemäß Strauß und Götz (Mol. Microbiol. 21:491-500, 1996) charakterisiert. Die molekulare Masse der Proteinstandards (in kDa) ist am linken Rand angegeben.

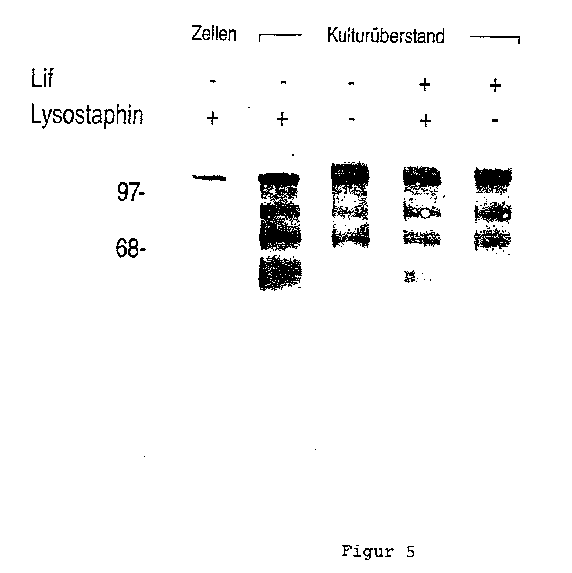

Figur 5 zeigt den Einfluß von Lysostaphin auf proLipFnBPB, das aus *S*. *carnosus* in das Kulturmedium durch natürliche Zellwandveränderungen freigesetzt wurde.
Überstände von Zellen, die proLipFnBPB (pTX30) in Gegenwart (+) oder Abwesenheit (-) von Lif (pCXlif) exprimieren, wurden in Gegenwart (+) oder Abwesenheit (-) von Lysostaphin untersucht. Als Referenz diente proLipFnBPB, das durch Lysostaphineinwirkung aus der Zellwand von *S*. *carnosus* Zellen, die nur das Plasmid pTX30 enthalten, freigesetzt wurde. Die Proteine wurden durch SDS-PAGE (10% Acrylamid) aufgetrennt und Immunoblotting (Prolipase-spezifisches Antiserum) durchgeführt. Die molekularen Massen der Standardmoleküle sind am linken Rand angegeben.

### Ausführungsbeispiel 1

### Simulationsszenarien

### Stämme & Plasmide

Wildtyp-Stamm *S. carnosus* TM300 (Götz, F., J. Appl. Bacteriol. Symp. Supp. 69: 49-53, 1990) wurde als Wirtsorganismus für die Herstellung aller rekombinanter Staphylokokkenstämme benutzt. Die Herstellung des Assay-Plasmids pTX30Δ82 wird nachfolgend beschrieben:

Plasmid pTX30Δ82 wurde analog zum Plasmid pCX30Δ82 (Strauß und Götz, Mol. Microbiol. 21:491-500, 1996) hergestellt. Es diente jedoch nicht das mit Chloramphenicol selektionierbare Plasmid pCX15 (Wieland et al., Gene 158: 91-96, 1995), sondern das mit Tetracyclin selektionierbare Plasmid pTX15 (Peschel et al., FEMS Microbiol. Lett. 137: 279-284, 1996) als Ausgangsvektor. Dieses Plasmid enthält eine Genfusion, welche das Assay-Hybridprotein (proLipFnBPBΔ82) bestehend aus *S*. *hyicus* Lipase und dem C-terminalen Teil des Fibronectin Bindeprotein B (FnBPB) kodiert und welche unter der Kontrolle des induzierbaren Xylose-Promotors steht (Wieland et al., Gene 158: 91-96, 1995). Der Abstand zwischen dem C-terminalen Alanin-Rest der Lipase und dem Leucin-Rest des LPXTG-Motives des FnBPB betrug 10 Aminosäuren (Figur 1). Die Plasmide pTX30Δ82.mem und pTX30Δ82.sec wurden analog zum Plasmid pTX30Δ82 unter Verwendung der Oligonukleotidpaare (Seq. ID No. 2 bis 5) AS14 (5'-ATAAGGCGCCTTAGTTTAATTATGCTTTGTGATTC)/AS45(5'-CGCAGGAAGCTT-ACCACAATCTAAGAAATCTGAAATATCTCAAGCAAGTGGAGAAG) und AS42(5'-AATAAGGCGCCTCATTATCCACCTGTTTCAGGTAGTTC)/AS22(5'-ACGAAAGCT-TACCACAATCTAAGAAATCTGAAC)ausgehend von pTX30 hergestellt. Die Plasmide pTX30Δ82, pTX30Δ82.mem und pTX30Δ82.sec wurden in *S*. *carnosus* TM300 transformiert (Götz & Schumacher, FEMS Microbiol. Lett. 40: 285-288, 1987). Die angegebenen Literaturstellen dienen der Ergänzung der Beschreibung.

### Medien

Für die Kultivierung der Bakterien in Flüssigkultur wurde Basis-Medium (BM) verwendet, das 1% Pepton, 0.5% Hefe-Extrakt, 0.5% NaCl, 0.1% Glucose und 0.1% Di-Kaliumhydrogenphosphat enthielt (pH 7.4). Für die Induktion des Xylose Promoters wurde modifiziertes Basis-Medium (Induktionsmedium) benutzt, in dem die Glucose durch 0,5% Xylose ersetzt wurde. Nach Bedarf wurde das BM mit Chloramphenicol (Cm, 10 mg/l), Tetracyclin (Tc, 25 mg/l) und Erythromycin (Em, 2.5 mg/l) versetzt. Agar-Selektionsplatten enthielten zusätzlich 15 g/l Agar. Lipase-Testplatten wurden unter Verwendung von Tributyrin-Agar Basis (Merck) nach Anleitung des Herstellers zubereitet. Der Agar wurde vor dem Gießen der Platten mit 1% Glycerintributyrat und den entsprechenden Antibiotika (Tc, Em) versetzt. Auf diesen Platten können Lipase-freisetzende Bakterienzellen durch die Bildung klarer Höfe identifiziert werden.

### Part I:

### Lokalisation und Quantifizierung der Lipase-Aktivität in den Simulations-Klonen

Jeweils 5 ml Basismedium wurden von Platte mit Wildtyp-Stamm *S*. *carnosus* TM300 bzw. den oben beschriebenen *S*. *carnosus* Klonen beimpft und bei 37°C über Nacht unter Schütteln inkubiert. Mit diesen Vorkulturen wurden jeweils 5 ml Induktionsmedium 1:100 beimpft und bei 37°C bis zur spät-logarithmischen Wachstumsphase geschüttelt. Die Kulturen wurden auf 4°C gekühlt, bevor die Lipase-Aktivität im Kulturüberstand und die zellgebundene Lipase-Aktivität nach Freisetzung durch Lysostaphinbehandlung der Zellen gemäß Strauß und Götz (Mol. Microbiol. 21: 491-500, 1996) bestimmt wurde. Die Gesamtaktivität ergab sich aus der Summe der zellgebundenen Aktivität und der Aktivität im Kulturüberstand. Anschließend wurde die jeweilige Lipase-Aktivität im Kulturüberstand in Relation zur Gesamtaktivität des entsprechenden Klons bestimmt (Figur 2). Die Literaturstelle dient der Ergänzung der Beschreibung.

### Part II:

### Etablierung eines auf Fluoreszenzspektroskopie basierenden Assayverfahrens

Die *S*. *carnosus* Klone *S*. *carnosus*/pTX30Δ82, /pTX30Δ82.mem, und /pTX30Δ82.sec wurden über Nacht bei 37°C in Basis-Medium kultiviert. Die Zelldichten dieser Kulturen wurden durch Messung der optischen Dichten (OD576) im Photometer bestimmt und Verdünnungen (ca. 1:200) gleicher Zelldichte hergestellt. Für die Herstellung dieser Verdünnungen wurde modifiziertes Basis-Medium (Induktionsmedium) verwendet. Die verdünnten Kulturen ließ man daraufhin erneut bei 37°C heranwachsen. In verschiedenen Experimenten wurden sowohl Mikrotiterplatten als auch andere Gefäße für die Kultivierungen verwendet.
Zu unterschiedlichen Zeitpunkten der Kultivierung wurde die von den Bakterien freigesetzte Lipaseaktivität in den Kulturüberständen bestimmt. Die Zellen wurden durch Zentrifugation sedimentiert, die Kulturüberstände abgehoben und, wenn notwendig, auf Eis aufbewahrt. Assays wurden in Mikrotiterplatten (100 *µ*l) mit Glasboden durchgeführt. Der Lipase-Assay-Puffer setzte sich wie folgt zusammen: 10 mM CaCl₂, 0,05% Triton-X-100, 20 mM Tris/HCl, pH 8,0. Als fluorogenes Farbsubstrat wurde 1,2-o-Dilauryl-rac-glycero-3-glutarsäure-resorufinester (Sigma # D7414) eingesetzt. Die Substrat-Stammlösung wurde in 100% DMSO bei 1mg/ml angesetzt und bei -20°C gelagert. Pro Messprobe wurden jeweils 10 *µ*l der Kulturüberstände mit 80 *µ*l Lipase-Assay-Puffer und 10 *µ*l Substrat-Lösung (10 *µ*M Endkonzentration) vermischt. Die Umsetzung des Substrats wurde fluorometrisch unter Verwendung eines Fluoreszenz-(ELISA)Readers oder mittels eines Fluoreszenz-Korrelationsspektrometers, wie dem ConfoCor™ (Carl-Zeiss-Jena, und Evotec, Deutschland) bestimmt.

Während der Klon *S*. *carnosus*/pTX30Δ82 in den Messungen nur unwesentlich höhere Signale als die Negativkontrolle (nur Puffer und Substrat) ergab, wurden in den Kulturüberständen der beiden Klone *S*. *carnosus*/pTX30Δ82.mem und *S*.*carnosus*/pTX30Δ82.sec signifikante Mengen an Lipaseaktivität gefunden.

### Ausführungsbeispiel 2:

### Stämme und Plasmide

Die nachfolgend angegebenen Literaturzitate dienen der Ergänzung der Beschreibung.

Der Wildtyp-Stamm S. *carnosus* TM300 (Götz, F., J. Appl. Bacteriol. Symp. Supp. 69: 49-53, 1990) wurde als Wirtsorganismus für die Herstellung aller rekombinanter Staphylokokkenstämme benutzt. Die Plasmide pTX15, pTX30 (kodiert ProLipFnBPB, ein Hybridprotein bestehend aus *S*. *hyicus* Lipase verbunden mit dem C-Terminus des S. *aureus* Fibronectin Bindungsproteins B) und pCXlif (lif, lysostaphin immunity factor) wurden in *S*. *carnosus* TM 300, wie von Götz und Schuhmacher (FEMS, Microbiol. Lett. 40:285-288, 1987) beschrieben, transformiert.

Die Konstruktion von pCXlif erfolgte gemäß des von Thumm und Götz (Mol. Microbiol. 23:1251-1256, 1997) beschriebenen Verfahrens.

Das Plasmid pTX30 wurde durch Insertion des *Bam*HI-*Nar*I Fragmentes von pCX30 (Strauß und Götz, Mol. Microbiol. 21:491-500, 1996) in das mit denselben Restriktionsenzymen geschnittene Plasmid pTX15 eingefügt.

Die Gene wurden unter Kontrolle des Xylose-Promotor-Systems exprimiert (Wieland et al., Gene 158:91-96,1995). Die Induktion der Expression erfolgte wie bei Strauß und Götz (Mol. Microbiol. 21:491-500, 1996) beschrieben.
Die Sequenzen der hierin verwendeten Nukleinsäuren sind wie folgt in Genbanken abgelegt: Genbank-Entry-Number: Plasmid pT181: g151679; *xyl*R (*S*. *xylosus*) : g48833; *lip* (*S. hyicus*) : g488333; Plasmid pC194: g150548; *fnb*B (*S*. *aureus*) : g49040.

### Medien

Die Bakterien wurden in Basis-Medium (BM; siehe Ausführungsbeispiel 1) bei 30°C kultiviert. Nach Bedarf wurde das Basismedium mit Chloramphenicol (Cm, 10mg/l) oder Tetracyclin (Tc, 25 mg/l) versetzt.

*Der Einfluß des Lysostaphin immunity factors (Lif) auf die Sekretion und die Verankerung von S. hyicus Lipase oder proLipFnBPB in der Zellwand durch Vergleich der Lipaseaktivität an der Zellwand und im Überstand des Kulturmediums*.

Die Zellkulturen (pTX15, pTX30, pTX15+pCXlif, pTX30+pCXlif) wurden zunächst durch Zentrifugation in Zellpellets und Medium getrennt. Anschließend wurden die Pellets dreimal mit BM gewaschen und in BM aufgenommen. Als Referenz dienten Zellwandproteine, die durch Behandlung mit Lysostaphin (80 *µ*g/ml in BM; 30 min bei 37°C) von pTX30 exprimierenden Zellen freigesetzt wurden. Von den Proben wurden Verdünnungen angefertigt. Hierzu wurden jeweils 5 *µ*l der Kulturüberstände mit 95 *µ*l Lipase-Assay-Puffer (10mM CaCl₂, 0.1% Triton X-100 und 20 mM Tris-HCl, pH 8.5), der das chromogene Lipasesubstrat p-Nitrophenyl Caprylat [Sigma] in einer Konzentration von 5 mM enthielt, versetzt. Die Hydrolyse des Substrates wurde anschließend über 10 Minuten bei 30°C photometrisch unter Verwendung eines Mikrotiterplatten (Elisa)-Readers (SpectraMax, Molecular Devices) oder mittels Fluoreszenz-Korrelationsspektroskopie mit dem ConfoCor bei einer Wellenlänge von 405 nm verfolgt. Die Assays wurden in Mikrotiterplatten ohne bzw. mit Glasboden durchgeführt.

Insgesamt zeigte sich, daß in Zellen, die pXT15 exprimieren die Gesamtlipaseaktivität im Überstand 99.2% und in Zellen die sowohl pXT15 als auch pCXlif exprimieren 99.1% beträgt. Als Kontrolle für die Verankerung der Lipase in der Zellwand dienten Zellen die pTX30 enthalten. Das pTX30 kodierte proLipFnBPB ist im Gegensatz zu z.B. pTX30Δ82-kodiertem proLipFnBPBΔ82 (siehe Ausführungsbeispiel 1) bei Verankerung in der Zellwand enzymatisch aktiv. In diesen Zellen lag die Gesamtlipaseaktivität an der Zelloberfläche bei 85.1%, wie auch bei Zellen, die sowohl pTX30 als auch pCXlif exprimieren (84.5%).

Es konnte somit gezeigt werden, daß die Lif Expression keinen Einfluß auf die Sekretion der Lipase oder die Verankerung von proLipFnBPB in der Zelloberfläche hat.

### Ausführungsbeispiel 3

### Stämme und Plasmide

Die verwendeten Stämme und Plasmide entsprechen den in Ausführungsbeispiel 2 genannten.

### Medien

Die Bakterien wurden in Basis-Medium (BM; siehe Ausführungsbeispiel 1) bei 30°C kultiviert. Nach Bedarf wurde das Basismedium mit Chloramphenicol (Cm, 10mg/l) oder Tetracyclin (Tc, 25 mg/l) versetzt.

### Bestimmung der enzymatischen Aktivität der freigesetzten Proteine

Es wurde die von den Bakterien freigesetzte Lipaseaktivität in den Kulturüberständen wie folgt bestimmt:
Die Zellen wurden durch Zentrifugation sedimentiert, die Kulturüberstände abgehoben und, wenn notwendig, auf Eis aufbewahrt. Assays wurden in Mikrotiterplatten ohne Glasboden durchgeführt. Der Lipase-Assay-Puffer (10mM CaCl₂, 0.1% Triton X-100 und 20 mM Tris-HCl, pH 8.5), enthielt das chromogene Lipasesubstrat p-Nitrophenyl Caprylat [Sigma] in einer Konzentration von 5 mM. Pro Messprobe wurden jeweils 5 *µ*l der Kulturüberstände mit 95 *µ*l Lipase-Assay-Puffer vermischt. Die Umsetzung des Substrats wurde photometrisch unter Verwendung eines Mikrotiterplatten (ELISA)-Readers bestimmt.

### Unterscheidung zwischen kovalent und nicht-kovalent an die Zellwand gebundenen Proteinen

Um auszuschließen, daß proLipFnBPB nicht-kovalent an die Zellwand der exprimierenden Zellen gebunden ist und aus diesem Grund bei der Bestimmung der enzymatischen Aktivität im Überstand nicht erfaßt wird, wurde eine Strategie entwickelt, die auf der Verwendung von Muramidase Ch und Lysostaphin basiert. Muramidase Ch hydrolysiert die β-1,4 Bindung von N-Acetylmuraminsäure und Acetylglucosamin (Figur 3) (Ghuysen, Bacteriol. Rev. 32: 425-464, 1968). Es schneidet nicht direkt an den Verknüpfungspunkten der Oberflächenproteine mit der Zellwand, so daß die Proteine mit Zellwandfragmenten variabler Länge abgespalten werden (Schneewind et al., EMBO J. 12: 4803-4811, 1993). Nicht-kovalent gebundene Proteine hingegen, die durch Muramidase Ch abgespalten werden, haben alle dieselbe molekulare Masse (Schneewind et al., EMBO J. 12: 4803-4811, 1993). Die angegebenen Literaturstellen dienen der Ergänzung der Beschreibung.

Die Zellen wurden aus 500 *µ*l Kulturmedium durch Zentrifugation gewonnen. Anschließend wurden die Pellets dann drei mal mit Wasser gewaschen und durch Zugabe von Trichloressäure (7% wt/vol) ausgefällt (20 min auf Eis). Nach der Zentrifugation des Präzipitates wurde das entstandene Pellet zweimal mit Azeton gewaschen und im Vakuum getrocknet. Die Pellets wurden dann in 170 *µ*l BM, dem Muramidase Ch (100 *µ*g ml⁻¹) zugesetzt wurde, gelöst und die Lösung für 3h bei 37°C inkubiert. Anschließend wurden die Proben erneut zentrifugiert und der Überstand in zwei Aliquots von je 80 *µ*l aufgeteilt, wovon eins mit 20 *µ*l Wasser, das andere mit 20 *µ*l Lysostaphin-Lösung (400 mg ml⁻¹) versetzt wurde. Die Lösungen wurden für 30 min bei 37°C inkubiert. Die einzelnen Aliquots wurden nun aufkonzentriert und unter Anwendung von SDS-PAGE (10% Acrylamid) und Immunoblotting (mit Prolipase spezifischem Antiserum) untersucht.

Es zeigte sich (Figur 4), daß Muramidase Ch zur vollständigen Freisetzung von proLipFnBPB aus der Zellwand von *S*. *carnosus* führt, unabhängig davon ob Lif (pCXlif) in diesen Zellen ebenfalls exprimiert wurde. In beiden Fällen war auf dem Gel ein Spektrum von Lipase-spezifischen Signalen als Schmier zu erkennen, der auf kovalent an proLipFnBPB gebundene Zellwandfragmente unterschiedlicher Länge zurückzuführen ist.

Zur Unterscheidung von Lif exprimierenden Zellen wurden die Proben in einer parallel durchgeführten Untersuchung vor Durchführung der Gelelektrophorese und des Immunoblottings mit Lysostaphin behandelt. Es zeigt sich, daß in den Fällen in denen proLipFnBPB aus Zellen, die kein Lif exprimieren, freigesetzt wurde, die Reste der Zellwandverankerung vollständig entfernt werden. Auf dem Gel war dies als exakte Bande zu erkennen. Oberflächenproteine, die aus Lif exprimierenden Zellen stammten, waren nicht Lysostaphin sensitiv (Figur 4).

### Ausführungsbeispiel 4

### Stämme und Plasmide

Die verwendeten Stämme und Plasmide entsprechen den in Ausführungsbeispiel 2 genannten.

### Medien

Die Bakterien wurden in Basis-Medium (BM; siehe Ausführungsbeispiel 1) bei 30°C kultiviert. Nach Bedarf wurde das Basismedium mit Chloramphenicol (Cm, 10mg/l) oder Tetracyclin (Tc, 25 mg/l) versetzt.

### Bestimmung der enzymatischen Aktivität der freigesetzten Proteine

Es wurde die von den Bakterien freigesetzte Lipaseaktivität in den Kulturüberständen gemäß Ausführungsbeispiel 3 bestimmt.

### Bestimmung des Anteils durch natürliche Zellwandveränderungen freigesetzter Oberflächenproteine aus S. carnosus.

Ein wichtiges Charakteristikum durch natürliche Zellwandveränderungen freigesetzter Zellwandproteine ist, daß sie vor der Freisetzung kovalent an die Zellwand gebunden sind. Zur Bestimmung des Anteils natürlich freigesetzter Proteine wird der Kulturüberstand von Zellen, die die Plasmide pCXLif und/oder pTX30 enthalten, aufkonzentriert und durch SDS-PAGE und Immunoblotting analysiert. Als Referenz diente proLipFnBPB das durch Lysostaphin aus Zellen, die nur das Plasmid pTX 30 enthalten, freigesetzt wurde. Es wurden zusätzlich zu Zersetzungsprodukten, die eine größere elektrophoretische Beweglichkeit als die Referenz besaßen, eine Summe von Lipase spezifischen Signalen, als Schmier beobachtet. Inkubation des Überstandes mit Lysostaphin (80 mg ml⁻¹ in BM, 30 min 37°C) vor Gelelektrophorese und Immunoblotting hatte nur einen Effekt auf Proteine, die aus dem Überstand von nicht-Lif exprimierenden Zellen gewonnen wurden. Statt dem Schmier aus einer Summe aus Lipasespezifischen Signalen ist eine begrenzte Bande zu sehen, die dieselbe elektrophoretische Beweglichkeit wie die Referenz zeigt. Die Lipasespezifischen Signale, die aus Zellen, die Lif und proLipFnBPB exprimieren stammen, werden nicht durch Lysostaphin beeinflußt.

In dieser Untersuchung zeigte sich darüber hinaus, daß durch natürliche Freisetzung insgesamt 5 % der Gesamtlipaseaktivität von Zellen, die ProLipFnBPB durch low-copy-number Plasmide exprimieren im Überstand gemessen wurde. Wohingegen bei medium-copy-number Plasmiden sogar 15% der Gesamtlipaseaktivität im Überstand zu bestimmen war. Die Koexpression anderer Oberflächenproteine hatte keinen Einfluß auf die Freisetzung der Lipase.

## Patentansprüche

1. Verfahren zur Bestimmung von Wirksubstanzen, welche die kovalente Verknüpfung von Polypeptiden an die Oberfläche von Gram-positiven Bakterien beeinflussen, mit folgenden Schritten:
a) Bereitstellen einer Probe Gram-positiver Bakterien, welche mindestens eine kovalent mit der Oberfläche der Gram-positiven Bakterien verbundene oder verbindbare enzymatische Reportersubstanz aufweisen oder bilden, wobei die mindestens eine Reportersubstanz ohne kovalente Bindung an die Oberfläche der Gram-positiven Bakterien eine andere enzymatische Aktivität aufweist als bei kovalenter Bindung an die Oberfläche der Gram-positiven Bakterien,
b) Inkontaktbringen der Probe mit einer möglichen Wirksubstanz, und
c) Bestimmen der enzymatischen Aktivität der Reportersubstanz der Gram-positiven Bakterien der Probe.

2. Verfahren nach Anspruch 1, dadurch gekennzeichnet, daß die enzymatische Aktivität der Reportersubstanz im Vergleich zu mindestens einer Referenzprobe, die nicht genetisch verändert wurde, und/oder mindestens einer Referenzprobe, in der die Reportersubstanz nicht-kovalent an die Oberfläche Gram-positiver Bakterien gebunden ist, und/oder mindestens einer Referenzprobe, in der die Reportersubstanz kovalent an die Oberfläche der Gram-positiven Bakterien gebunden ist, und/ oder mindestens einer Referenzprobe, in der die Reportersubstanz ohne kovalente Bindung an die Oberfläche der Gram-positiven Bakterien vorliegt, bestimmt wird.

3. Verfahren nach Ansprüchen 1 und/oder 2, dadurch gekennzeichnet, daß die kovalente Verknüpfung der Polypeptide an das Murein der Zellwand, insbesondere an Interpeptidbrücken wie z.B. Pentaglycine, Gram-positiver Bakterien erfolgt.

4. Verfahren nach mindestens einem der Ansprüche 1 bis 3,
dadurch gekennzeichnet, daß die Polypeptide Pathogenitätsfaktoren Gram-positiver Bakterien darstellen.

5. Verfahren nach mindestens einem der Ansprüche 1 bis 4,
dadurch gekennzeichnet, daß die Reportersubstanz ein Hybridpolypeptid ist.

6. Verfahren nach Anspruch 5, dadurch gekennzeichnet, daß das Hybridpolypeptid eine Anordnung der folgenden Sequenzabschnitte aufweist: N-terminales Signalpeptid, Enzym, Sequenzabschnitt mit der Sequenz LPXTG, hydrophober Sequenzabschnitt und geladener Sequenzabschnitt.

7. Verfahren nach Anspruch 6, dadurch gekennzeichnet, daß das Enzym als Proenzym bereitgestellt wird.

8. Verfahren nach mindestens einem der Ansprüche 6 bis 7,
dadurch gekennzeichnet, daß die Änderung der enzymatischen Aktivität durch einen Übergang des Enzyms aus einer inaktiven in eine aktive Konformation oder umgekehrt bedingt ist.

9. Verfahren nach Anspruch 6 bis 8, dadurch gekennzeichnet, daß zwischen Enzym und Sequenzabschnitt mit der Sequenz LPXTG ein Linkerpeptid, welches insbesondere weniger als 10 Aminosäuren umfaßt, angeordnet ist.

10. Verfahren nach mindestens einem der Ansprüche 1 bis 9,
dadurch gekennzeichnet, daß die Gram-positiven Bakterien einen geringen natürlichen Zellwand turn-over und/oder eine geringe Anzahl an Zellwandproteasen und/oder eine geringe Anzahl an sekretierten Proteasen aufweisen.

11. Verfahren nach mindestens einem der Ansprüche 1 bis 10,
dadurch gekennzeichnet, daß die Bestimmung der enzymatischen Aktivität der mindestens einen Reportersubstanz mittels Fluoreszenzspektroskopie, insbesondere konfokaler Fluoreszenzspektroskopie erfolgt.

12. Verfahren nach mindestens einem der Ansprüche 1 bis 11,
dadurch gekennzeichnet, daß die Gram-positiven Bakterien Lif exprimieren.

13. Verfahren nach mindestens einem der Ansprüche 1 bis 12,
dadurch gekennzeichnet, daß der Anteil der durch natürliche Zellwandveränderungen freigesetzten Reportersubstanzen bestimmt wird.

14. Verfahren nach mindestens einem der Ansprüche 1 bis 13,
dadurch gekennzeichnet, daß der Anteil nicht-kovalent mit der Oberfläche Gram-positiver Bakterien verbundener Reportersubstanzen bestimmt wird.

## Claims

1. A method for identifying active substances which affect the covalent bonding of polypeptides to the surface of Gram-positive bacteria, comprising the following steps:
a) providing a sample of Gram-positive bacteria which contain or produce at least one enzymatic reporter substance which is or can become covalently bonded to the surface of the Gram-positive bacteria, said at least one reporter substance having a different enzymatic activity when not covalently bonded to the surface of the Gram-positive bacteria from that exhibited when it is covalentfy bonded to the surface of the Gram-positive bacteria;
b) contacting the sample with a possible active substance; and
c) assaying the enzymatic activity of the reporter substance of the Gram-positive bacteria of the sample.

2. The method according to claim 1, characterized in that said assaying of the enzymatic activity of the reporter substance is done by comparison with at least one reference sample which has not been genetically altered, and/or at least one reference sample in which the reporter substance is non-covalently bonded to the surface of Gram-positive bacteria, and/or at least one reference sample in which the reporter substance is covalently bonded to the surface of the Gram-positive bacteria, and/or at least one reference sample in which the reporter substance is present without covalent bonding to the surface of the Gram-positive bacteria.

3. The method according to claims 1 and/or 2, characterized in that said covalent bonding of the polypeptides is effected to the murein of the cell wall, especially at interpeptide bridges, such as pentaglycines, of Gram-positive bacteria.

4. The method according to at least one of claims 1 to 3, characterized in that said polypeptides are pathogenicity factors of Gram-positive bacteria.

5. The method according to at least one of claims 1 to 4, characterized in that said reporter substance is a hybrid polypeptide.

6. The method according to claim 5, characterized in that said hybrid polypeptide has a succession of the following sequence segments: N-terminal signal peptide, enzyme, sequence segment having the sequence LPXTG, hydrophobic sequence segment, and charged sequence segment.

7. The method according to claim 6, characterized in that said enzyme is provided as a proenzyme.

8. The method according to at least one of claims 6 to 7, characterized in that said change of enzymatic activity is due to a transition of the enzyme from an inactive to an active conformation or vice versa.

9. The method according to claim 6 to 8, characterized in that a linker peptide, especially one comprising less than 10 amino acids, is provided between said enzyme and said sequence segment having the sequence LPXTG.

10. The method according to at least one of claims 1 to 9, characterized in that said Gram-positive bacteria have a low natural cell wall turnover and/or a small number of cell wall proteases and/or a small number of secreted proteases.

11. The method according to at least one of claims 1 to 10, characterized in that said assaying of the enzymatic activity of said at least one reporter substance is done using fluorescence spectroscopy, especially confocal fluorescence spectroscopy.

12. The method according to at least one of claims 1 to 11, characterized in that said Gram-positive bacteria express Lif.

13. The method according to at least one of claims 1 to 12, characterized in that the fraction of reporter substances released by natural cell wall changes is determined.

14. The method according to at least one of claims 1 to 13, characterized in that the fraction of reporter substances which are non-covalently bonded to the surface of Gram-positive bacteria is determined.

## Revendications

1. Procédé pour déterminer des matières actives qui influent sur la liaison covalente de polypeptides à la surface de bactéries Gram-positives, avec les étapes suivantes :
a) mise à disposition d'un échantillon de bactéries Gram-positives, qui comportent ou forment au moins une substance reporter enzymatique, liée ou pouvant être liée d'une manière covalente à la surface des bactéries Gram-positives, où au moins une substance reporter sans liaison covalente à la surface des bactéries Gram-positives présente une activité enzymatique différente de celle correspondant à une liaison covalente à la surface des bactéries Gram-positives,
b) mise en contact de l'échantillon avec une éventuelle matière active, et
c) détermination de l'activité enzymatique de la substance reporter des bactéries Gram-positives de l'échantillon.

2. Procédé selon la revendication 1, caractérisé en ce que l'activité enzymatique de la substance reporter est déterminée par comparaison à au moins un échantillon de référence qui n'a pas subi de modification génétique, et/ou à au moins un échantillon de référence dans lequel la substance reporter est liée d'une manière non-covalente à la surface de bactéries Gram-positives, et/ou à au moins un échantillon de référence dans lequel la substance reporter est liée d'une manière covalente à la surface des bactéries Gram-positives, et/ou à au moins un échantillon de référence dans lequel la substance reporter est présente sans liaison covalente à la surface des bactéries Gram-positives.

3. Procédé selon les revendications 1 et/ou 2, caractérisé en ce que la liaison covalente des polypeptides a lieu à la muréine de la paroi cellulaire, en particulier aux ponts interpeptidiques, tels par exemple que les pentaglycines, de bactéries Gram-positives.

4. Procédé selon au moins l'une des revendications 1 à 3, caractérisé en ce que les polypeptides représentent des facteurs de pathogénicité de bactéries Gram-positives.

5. Procédé selon au moins l'une des revendications 1 à 4, caractérisé en ce que la substance reporter est un polypeptide hybride.

6. Procédé selon la revendication 5, caractérisé en ce que le polypeptide hybride comporte les motifs de séquence ayant la disposition suivante : peptide signal N-terminal, enzyme, motif de séquence ayant la séquence LPXTG, motif de séquence hydrophobe et motif de séquence chargé.

7. Procédé selon la revendication 6, caractérisé en ce que l'enzyme est mise à disposition sous forme d'une pro-enzyme.

8. Procédé selon au moins l'une des revendications 6 et 7, caractérisé en ce que la modification de l'activité enzymatique est provoquée par une transition de l'enzyme, d'une structure inactive à une structure active ou inversement.

9. Procédé selon les revendications 6 à 8, caractérisé en ce qu'un peptide lieur, qui comporte en particulier moins de 10 acides aminés, est disposé entre l'enzyme et le motif de séquence ayant la séquence LPXTG.

10. Procédé selon au moins l'une des revendications 1 à 9, caractérisé en ce que les bactéries Gram-positives présentent un faible taux de renouvellement naturel de la paroi cellulaire et/ou un petit nombre de protéases de la paroi cellulaire et/ou un petit nombre de protéases secrétées.

11. Procédé selon au moins l'une des revendications 1 à 10, caractérisé en ce que la détermination de l'activité enzymatique d'au moins une substance reporter a lieu par spectroscopie de fluorescence, en particulier par spectroscopie de fluorescence confocale.

12. Procédé selon au moins l'une des revendications 1 à 11, caractérisé en ce que les bactéries Gram-positives expriment le Lif.

13. Procédé selon au moins l'une des revendications 1 à 12, caractérisé en ce qu'on détermine la proportion des substances reporter libérées par des modifications naturelles de la paroi cellulaire.

14. Procédé selon au moins l'une des revendications 1 à 13, caractérisé en ce qu'on détermine la proportion de substances reporter liées d'une manière non-covalente à la surface de bactéries Gram-positives.
